# EUROPÄISCHE PATENTSCHRIFT

(11) **EP 1 553 959 B1**
(45) Veröffentlichungstag und Bekanntmachung des Hinweises auf die Patenterteilung: **08.04.2009**
(21) Anmeldenummer: 03808742.5
(22) Anmeldetag: 08.10.2003
(51) Int. Cl.: A61K 35/22, A61K 38/22

(54) **VERFAHREN ZUR GEWINNUNG EINES AN NICHT-KONJUGIERTEN LIPOPHILEN VERBINDUNGEN ABGEREICHERTEN NAT RLICHEN GEMISCHES KONJUGIERTER EQUINER STROGENE**
METHOD FOR EXTRACTING A NATURAL MIXTURE OF CONJUGATED EQUINE ESTROGENS THAT IS DEPLETED OF NON-CONJUGATED LIPOPHILIC COMPOUNDS
PROCEDE D'EXTRACTION D'UN MELANGE NATUREL D'OESTROGENES EQUINS CONJUGUES APPAUVRI EN COMPOSES LIPOPHILES NON CONJUGUES

(30) Priorität: 11.10.2002 EP 02022763
(43) Veröffentlichungstag der Anmeldung: 20.07.2005
(73) Patentinhaber: Solvay Pharmaceuticals GmbH, 30173 Hannover (DE)
(72) Erfinder: BAN, Ivan, 30539 Hannover (DE); GERLING, Klaus-Guenther, 29308 Winsen/Aller (DE); MUELLER, Hans-Joerg, 30627 Hannover (DE); WACHSMANN, Stefan, 30167 Hannover (DE)
(74) Vertreter: Gosmann, Martin
(86) Internationale Anmeldenummer: PCT/EP2003/050703
(87) Internationale Veröffentlichungsnummer: WO 2004/035067

(56) Entgegenhaltungen:
- WO-A-98/08525
- US-A- 2 551 205
- US-A- 2 565 115

## Beschreibung

Die vorliegende Erfindung betrifft die Gewinnung eines natürlichen Gemisches konjugierter equiner Östrogene, welches an nicht-konjugierten lipophilen Verbindungen aus der Gruppe umfassend nicht-konjugierte Flavonoide, nicht-konjugierte Isoflavonoide, nicht-konjugierte Norisoprenoide, nicht-konjugierte Steroide, insbesondere Androstan- und Pregnan-Steroide, und vergleichbare nicht-konjugierte Verbindungen, abgereichert ist.

Östrogene werden in der Medizin zur Hormonsubstitutionstherapie eingesetzt. Insbesondere werden Östrogengemische zur Behandlung und Prophylaxe der, bei Frauen auftretenden Beschwerden der Wechseljahre nach natürlicher oder artifizieller Menopause eingesetzt. Hierbei haben sich natürliche Gemische konjugierter Östrogene, wie sie im Harn trächtiger Stuten vorliegen, im Folgenden als natürliche Gemische konjugierter equiner Östrogene bezeichnet, als besonders wirksam und gut verträglich erwiesen.

Der gelöste Feststoffgehalt im Harn trächtiger Stuten (= pregnant mares urine, im Folgenden abgekürzt als "PMU") kann natürlicherweise in weiten Bereichen schwanken und im Allgemeinen in einem Bereich von 40 bis 90 g Trockensubstanz pro Liter liegen. Neben Harnstoff und sonstigen üblichen Harninhaltsstoffen sind in dem Feststoffgehalt des PMU phenolische Bestandteile in Mengen von ca. 2 bis 5 Gew.-% bezogen auf die Trockensubstanz enthalten. Unter diesen phenolischen Bestandteilen befinden sich Kresole und das als HPMF bekannte Dihydro-3,4-bis[(3-hydroxyphenyl)methyl]-2(3H)-furanon. Diese können in freier oder konjugierter Form vorliegen. In dem PMU ist ein natürliches Gemisch von Östrogenen enthalten, welches weitgehend in konjugierter Form, z. B. als Schwefelsäurehalbester-Natriumsalz (im folgenden abgekürzt als "Sulfatsalz"), vorliegt. Der Gehalt an konjugierten Östrogenen (berechnet als Östrogensulfatsalz) kann zwischen 0,1 und 1 Gew.-% bezogen auf die Trockensubstanz betragen. Zusätzlich können im Feststoffgehalt des PMU weitere lipophile Verbindungen vorliegen, deren Mengen in weiten Bereichen schwanken können und nicht vorhersehbar sind. Diese lipophilen Verbindungen stammen überwiegend aus den von den trächtigen Stuten als Nahrung aufgenommen Pflanzen und umfassen vor allem verschiedene Flavonoid-, Isoflavonoid-, Norisoprenoid-Derivate und vergleichbare Verbindungen, wie beispielsweise Formononetin, Genistein, Daidzein, Biochanin A, Equol und Coumestrol. Diese lipophilen Verbindungen ursprünglich pflanzlicher Herkunft können im Harn in konjugierter oder in freier (nicht konjugierter) Form vorliegen. Zu den, im Feststoffgehalt des PMU weiterhin vorkommenden lipophilen Bestandteilen zählen auch nicht-konjugierte Steroid-Derivate, hier seien insbesondere die Androstan- und Pregnan-Steroide, aber auch nicht-konjugierte Östrogen-Derivate genannt.

Natürliche Gemische konjugierter Östrogene enthaltende Extrakte werden üblicherweise entweder mittels eines Festphasen-Extraktionsverfahren oder durch ein Verfahren gewonnen, das auf verschiedenen Flüssig-Flüssig-Extraktionsschritten mit, mit Wasser nicht oder nur wenig mischbaren organischen Lösemitteln basiert. Allgemein gilt, dass das gewonnene natürliche Gemisch konjugierter Östrogene, um als Wirkstoffkomponente für Pharmazeutika verwendet werden zu können, bestimmte pharmazeutische Spezifikationen erfüllen muss, beispielsweise muss es der in der USP (United States Pharmacopeia) oder European Pharmacopeia festgelegten Spezifikation entsprechen. So müssen hinsichtlich des Gehalts an konjugierten Östrogenen bezogen auf die Trockensubstanz bestimmte Grenzwerte eingehalten werden.

Die US Patente No. 2,551,205 und No. 2,429,398 beschreiben ein Verfahren zur Herstellung einer wasser-löslichen Östrogen-Präparation aus PMU, bei dem zunächst durch Adsorption an Aktivkohle oder andere geeignete Adsorbermaterialien, Elution mit einem mit Wasser mischbaren organischen Lösungsmittel, wie z. B. Pyridin, und anschließende Entfernung des Lösungsmittels ein wässriges Konzentrat erhalten wird, welches den größten Teil der wasserlöslichen Östrogen-Bestandteile des ursprünglich eingesetzten PMUs enthält. Während im US Patent No. 2,429,398 das Konzentrat durch Extraktion mit Benzol und / oder Ether weiter aufgereinigt wird, wird im US Patent No. 2,551,205 beschrieben, dass das Konzentrat auf einen pH-Wert zwischen 2 und 6, vorzugsweise zwischen 4 und 5, angesäuert und dann schnell mit einem mit Wasser kaum mischbaren organischen Lösungsmittel aus der Gruppe der aliphatischen, aromatischen oder alizyklischen Kohlenwasserstoffe (z. B. Hexan, Benzol, Toluol, Cyclohexan) oder der chlorierten Kohlenwasserstoffe (z. B. Chloroform, Ethylendichlorid, Trichlorethylen, Tetrachlorkohlenstoff, Chlorbenzol) extrahiert wird, um unerwünschte Substanzen wie Fette, Öle, freie phenolische Bestandteile und die nicht-konjugierten Steroide durch Überführung in die organische Phase abzutrennen. Abschließend wird die wässrige Phase durch Neutralisation stabilisiert. Das US Patent No. 2,551,205 empfiehlt, den gewonnenen Extrakt durch nachfolgende Extraktionsschritte und Präzipitationen weiter aufzureinigen. Insgesamt wird nach Durchführung des, im US Patent No. 2,551,205 beschriebenen Verfahrens nur eine Ausbeute von etwa 80 % der Östrogen-Bestandteile des eingesetzten Konzentrates erhalten.

Im US Patent No. 2,565,115 wird die Extraktion der konjugierten Östrogene aus PMU mit Aceton beschrieben. Über die Reinheit der erhaltenen Östrogenfraktion wird keine Aussage gemacht.

Das US Patent No. 2,696,265 beschreibt ein Verfahren, in dem zunächst die Östrogene mit einem aliphatischen Alkohol oder Keton, wie Hexanol, Cyclohexanol oder Cyclohexanon, extrahiert werden. Die Östrogene treten in die organische Phase über und werden anschließend weiter aufgereinigt, u.a. wird eine die Östrogene enthaltende wässrige Phase mit Salzsäure auf einen pH-Wert von 4 eingestellt und mit Ethylendichlorid extrahiert.

Im US Patent Nr. 2,834,712 wird ein Verfahren zur Herstellung von Östrogengemischen signifikanter Reinheit und geringer Toxizität offenbart, das auf einer Vielzahl einzelner Extraktionsschritte mit unterschiedlichen Lösungsmitteln und bei Einstellung verschiedener pH-Werte beruht. Dabei finden große Volumina an Lösungsmitteln wie Hexan und Benzol Verwendung. So wird z. B. in einem Schritt ein bereits aufgereinigtes Konzentrat in Wasser gelöst, mit Salzsäure auf einen pH-Wert von circa 5.0 eingestellt und mit Benzol und anschließend mit Ether extrahiert, um die phenolischen Bestandteile abzutrennen.

Die internationale Patentanmeldung WO 01/27134 beschreibt ein vergleichsweise einfaches Verfahren, um konjugierte Östrogene aus PMU zu extrahieren: Nach Zugabe eines Salzes, wie z. B. Natriumchlorid, wird der PMU mit mindestens dem gleichen Volumenanteil eines organischen Lösungsmittels, wie z. B. Ethylacetat, extrahiert, dabei treten die konjugierten Östrogene in die organische Phase über. Die organische Phase wird abgetrennt und getrocknet, um den Extrakt zu erhalten. In der WO 01/27134 werden keine Aussagen über die Reinheit des erhaltenen Extraktes konjugierter Östrogene gemacht.

Bei den vorstehend beschriebenen und aus dem Stand der Technik bekannten Flüssig-Flüssig-Extraktionsverfahren treten jedoch eine Vielzahl von Problemen, wie starke Schaumbildung, Sedimentbildung, Emulsionsbildung und schlechte Phasentrennung auf. Es werden im allgemeinen mehrere Extraktionsschritte benötigt, was zu Verlusten und nur teilweisen Gewinnung des Östrogengehaltes führt. Weiterhin erfordern diese Extraktionsverfahren große Volumina an zum Teil gesundheitsschädlichen Lösungsmitteln. Weiterhin werden in den oben angeführten Patentschriften weder über den Gehalt an nicht-konjugierten lipophilen Bestandteilen, wie beispielsweise nicht-konjugierte Flavonoid-, Isoflavonoid-, Norisoprenoid-Derivate und vergleichbare nicht-konjugierte Verbindungen, oder auch nicht-konjugierte Steroide, insbesondere Androstan- und Pregnan-Steroide, in den erhaltenen Produkten noch über eine Abtrennung dieser Bestandteile Aussagen gemacht. Diese aus dem Stand der Technik bekannten Verfahren liefern entweder keine zufriedenstellenden Ergebnisse hinsichtlich der Ausbeute oder hinsichtlich der Reinheit des erhaltenen Extraktes, gemessen am erhaltenen Gesamthormongehalt bezogen auf die Trockensubstanz, oder sie beruhen auf einer Vielzahl verschiedener Verfahrensschritte und der Verwendung großer Volumina organischer und z. T. sogar aus toxikologischer Sicht unerwünschter Lösungsmittel.

Weiterhin sind aus dem Stand der Technik verschiedene Festphasen-Extraktionsverfahren bekannt, um ein, an phenolischen Harninhaltsstoffen weitgehend abgereichertes, natürliches Gemisch konjugierter equiner Östrogene zu gewinnen. So beschreibt die internationale Patentanmeldung WO 98/08526 ein Verfahren, mit welchem in einer Festphasen-Extraktion an einem semipolaren, insbesondere an einem nichtionischen semipolaren, polymeren Adsorberharz ein, an phenolischen Harninhaltsstoffen abgereichertes, weitgehend Kresol- und HPMF-freies, den natürlichen Östrogen-Gehalt des PMU praktisch vollständig enthaltendes Gemisch gewonnen werden kann. In der internationalen Patentanmeldung WO 98/08525 wird ein ähnliches Verfahren beschrieben, in dem bei der Festphasen-Extraktion Kieselgel als Adsorbermaterial zum Einsatz kommt. Auch die chinesische Patentanmeldung CN 1308083 beschreibt ein vergleichbares Verfahren, bei dem Cyanogruppenhaltige polare Adsorberharze verwendet werden. Die erhaltenen Extrakte eignen sich als Ausgangsmaterial für die Herstellung von Pharmazeutika, welche als Wirkstoffkomponente das natürliche Gemisch konjugierter Östrogene aus PMU enthalten.

Die gestellten pharmazeutischen Spezifikationsanforderungen, beispielsweise die hinsichtlich des Gehalts an konjugierten Östrogenen bezogen auf die Trockensubstanz einzuhaltenden Grenzwerte, werden normalerweise von den aus PMU gemäß dem Verfahren der WO 98/08526 oder dem Verfahren der WO 98/08525, gewonnenen Gemischen konjugierter Östrogene erfüllt. Es hat sich aber herausgestellt, dass neben dem gewünschten Gehalt an konjugierten Östrogenen auch nicht-konjugierte lipophile Verbindungen in der gewonnenen Trockensubstanz enthalten sein können. Zu den nicht-konjugierten lipophilen Verbindungen zählen beispielsweise verschiedene nicht-konjugierte Flavonoid-, Isoflavonoid-, Norisoprenoid-Derivate und vergleichbare nicht-konjugierte Verbindungen, wie beispielsweise Formononetin, Genistein, Daidzein, Biochanin A, Equol und Coumestrol, aber auch nicht-konjugierte Steroide, insbesondere Androstan- und Pregnan-Steroide, und nicht-konjugierte Östrogene, wobei diese Aufzählung nicht als abschließend zu betrachten ist. Die Anwesenheit der nicht-konjugierten lipophilen Verbindungen im aus dem PMU gewonnenen Gemisch konjugierter Östrogene lässt sich nicht standardisieren, sondern sowohl der Gehalt als auch die Zusammensetzung der freien und der konjugierten lipophilen Verbindungen variiert beispielsweise in Abhängigkeit von der aufgenommenen Nahrung der trächtigen Stuten.

Zwar verändert sich die Zusammensetzung des natürlichen Gemisches an konjugierten equinen Östrogenen durch die zusätzliche Anwesenheit der nicht-konjugierten lipophilen Verbindungen nicht, aber der Gehalt der konjugierten equinen Östrogene bezogen auf die Trockensubstanz kann vermindert sein. Durch eine gezielte Abtrennung der nicht-konjugierten lipophilen Bestandteile könnte eine höhere Konzentration der Wirksubstanzen, d.h. der konjugierten equinen Östrogene, im erhaltenen Extrakt erreicht werden. Auch aus Gründen der Arzneimittelsicherheit kann es sinnvoll sein, die nicht-konjugierten lipophilen Verbindungen abzutrennen, um eine gleichmäßige Zusammensetzung einzelner Extraktchargen zu gewährleisten, da so die nicht-konjugierten lipophilen Bestandteile, deren Gehalt und Zusammensetzung im PMU in Abhängigkeit von der jahreszeitlich wechselnden Art der aufgenommenen Nahrung der trächtigen Stuten variieren kann, eliminiert werden, und damit die erhaltenen Extrakte alle einen vergleichbaren Gehalt konjugierter equiner Östrogene bezogen auf die Trockensubstanz aufweisen würden. Weiterhin kann eine Abtrennung der nicht-konjugierten lipophilen Verbindungen vorteilhaft sein, um ein gleichmäßiges physiologisches Wirkungsspektrum zu erzielen. Beispielsweise kann es sinnvoll sein, gegebenenfalls anwesende, nicht-konjugierte lipophile Verbindungen, die möglicherweise selbst eine unerwünschte physiologische Wirkung besitzen können, aus dem natürlichen Gemisch konjugierter equiner Östrogene abzutrennen.

Aufgabe der vorliegenden Erfindung ist es daher, ein technisch und wirtschaftlich optimales Verfahren zur Gewinnung eines natürlichen Gemisches konjugierter equiner Östrogene zu entwickeln, wobei das Gemisch an nicht-konjugierten lipophilen Verbindungen, insbesondere an nicht-konjugierten Flavonoid-, Isoflavonoid- und Norisoprenoid-Derivaten weitgehend abgereichert ist. Insbesondere ist es die Aufgabe der vorliegenden Erfindung ein solches Verfahren zu entwickeln, bei dem nur geringe Mengen nicht gesundheitsschädlicher Lösungsmittel eingesetzt werden. Weiterhin soll ein Verfahren entwickelt werden, das nur auf wenigen Verfahrensschritten beruht und dabei einen Extrakt konjugierter equiner Östrogene liefert, welcher einen vergleichsweise hohen Gehalt an konjugierten Östrogenen bezogen auf die Trockensubstanz aufweist. Weiterhin soll es mit dem Verfahren der vorliegenden Erfindung auf einfache Weise möglich sein, ein bereits an phenolischen Harninhaltstoffen abgereichtes Gemisch konjugierter Östrogene aus dem Harn trächtiger Stuten, welches wechselnde und gegebenenfalls erhöhte Mengen an nicht-konjugierten lipophilen Verbindungen enthalten kann, so zu behandeln, dass das mit der vorliegenden Erfindung gewonnene natürliche Gemisch konjugierter equiner Östrogene gute Wirkstoffgehalte aufweist und die geforderten pharmazeutischen Spezifikationen erfüllt, insbesondere soll es die erforderlichen Grenzwerte hinsichtlich des Gehalts an konjugierten Östrogenen bezogen auf die Trockensubstanz einhalten.

Es wurde nun ein Verfahren gefunden, mit welchem auf überraschend einfache Weise ein Gemisch konjugierter equiner Östrogene auch aus einem wechselnde und gegebenenfalls erhöhte Mengen an nicht-konjugierten lipophilen Verbindungen aufweisenden PMU gewonnenen werden kann, wobei das gewonnene Gemisch konjugierter equiner Östrogene an nicht-konjugierten lipophilen Verbindungen, insbesondere an nicht-konjugierten Flavonoid-, Isoflavonoid- und Norisoprenoid-Derivaten weitgehend abgereichert ist. Insbesondere kann das erfindungsgemäße Verfahren Anwendung finden auf ein bereits an phenolischen Harninhaltstoffen abgereichtes Gemisch konjugierter Östrogene aus dem Harn trächtiger Stuten, so dass mit dem Verfahren ein Gemisch konjugierter equiner Östrogene gewonnen wird, welches eine hohe Produktqualität aufweist und die Anforderungen der pharmazeutischen Spezifikation, insbesondere auch im Hinblick auf die einzuhaltenden Grenzwerte hinsichtlich des Gehalts an konjugierten Östrogenen bezogen auf die Trockensubstanz, sicher erfüllt.

Das erfindungsgemäße Verfahren zur Gewinnung eines natürlichen Gemisches konjugierter equiner Östrogene, ist **dadurch gekennzeichnet, dass** das gewonnene Gemisch abgereichert ist an nicht-konjugierten lipophilen Verbindungen aus der Gruppe umfassend nicht-konjugierte Flavonoide, nicht-konjugierte Isoflavonoide, nicht-konjugierte Norisoprenoide, nicht-konjugierte Steroide, insbesondere Androstan- und Pregnan-Steroide, und vergleichbare nicht-konjugierte Verbindungen, und dass das Verfahren sich dadurch auszeichnet, dass man
a) eine wässrige Ausgangsphase ausgewählt aus der Gruppe umfassend
   (i) eine wässrige Lösung eines an phenolischen Harninhaltsstoffen bereits abgereicherten natürlichen Gemisches konjugierter Östrogene aus dem Harn trächtiger Stuten,
   (ii) ein wässriges Konzentrat eines an phenolischen Harninhaltsstoffen bereits abgereicherten natürlichen Gemisches konjugierter Östrogene aus dem Harn trächtiger Stuten,
   (iii) ein durch Einengen gewonnenes Konzentrat einer Harnflüssigkeit, und
   (iv) ein von Fest- und Schleimstoffen befreites Harnkonzentrat, welches durch Trennverfahren ausgewählt aus der Gruppe umfassend Dekantieren, Separieren, Filtrieren und Kombinationen der vorgenannten Verfahren vorgereinigt wurde, oder ein durch Filtration gewonnenes aufkonzentriertes Harn-Retentat,
      einer Flüssig-Flüssig Extraktion mit einer ausreichenden Menge eines Extraktionsmittels unterwirft und im Anschluß die wäßrige Phase abtrennt, wobei das Extraktionsmittel, ein, für die Extraktion von nicht-konjugierten lipophilen Verbindungen aus oben angegebener Gruppe geeignetes, mit Wasser nicht oder kaum mischbares organisches Lösungsmittel darstellt und ausgewählt ist aus der Gruppe bestehend aus geradkettigen, verzweigten oder zyklischen C₄-C₁₀ Alkoholen, C₂-C₁₀ veresterten Säuren, C₃-C₁₀ Aldehyden, C₄-C₁₀ Ketonen, C₂-C₁₀ Ethern, C₃-C₆ Nitrilen, und C₁-C₃ Halogenalkanen, und Mischungen der vorgenannten Lösungsmittel, und
b) gegebenenfalls mit der erhaltenen wässrigen Phase den Verfahrensschritt (a) wiederholt, und
c) eine das natürliche Gemisch konjugierter Östrogene enthaltende wässrige Phase gewinnt und diese gegebenenfalls einengt.

Für das erfindungsgemäße Verfahren können als wässrige Ausgangsphase eine wässrige Lösung (i) eines an phenolischen Harninhaltsstoffen bereits abgereicherten natürlichen Gemisches konjugierter Östrogene aus dem Harn trächtiger Stuten oder ein wässriges Konzentrat (ii) eines an phenolischen Harninhaltsstoffen bereits abgereicherten natürlichen Gemisches konjugierter Östrogene aus dem Harn trächtiger Stuten eingesetzt werden. Diese wässrige Lösung oder dieses wässrige Konzentrat können durch ein Verfahren erhalten werden, wie es beispielsweise bereits in den internationalen Patentanmeldungen WO 98/08526 und WO 98/08525 oder in der chinesischen Patentanmeldung CN 1308083 beschrieben worden und dem Fachmann somit aus diesen publizierten Patentanmeldungen geläufig ist. Der Inhalt dieser WO 98/08526, WO 98/08525 und CN 1308083 wird zum Zwecke der Offenbarung auch zum Gegenstand der vorliegenden Anmeldung gemacht. Eine wässrige Lösung oder ein wässriges Konzentrat eines an phenolischen Harninhaltsstoffen bereits abgereicherten natürlichen Gemisches konjugierter Östrogene aus dem Harn trächtiger Stuten kann auch das Produkt eines Flüssig-Flüssig-Extraktionsprozesses sein, wie er beispielsweise in der internationalen Patentanmeldung WO 01/27134 beschrieben wird. Die nach den in den vorstehenden Patentanmeldungen beschriebenen Verfahren erhaltenen wässrigen Lösungen oder Konzentrate können vor ihrer Verwendung im erfindungsgemäßen Verfahren auf an sich bekannte Weise, wie beispielsweise Destillation, weiter eingeengt werden, um ein weitgehend von organischem Lösemittel befreites Konzentrat zur erhalten.

Weiterhin können für das erfindungsgemäße Verfahren als wässrige Ausgangsphase ein aus dem PMU durch Einengen gewonnenes Konzentrat (iii) oder ein aus dem PMU gewonnenes Konzentrat (iv), welches durch Filtration oder vergleichbare Verfahren bereits vorgereinigt wurde, eingesetzt werden. Der gesammelte Harn (PMU) wird zunächst auf an sich bekannte Weise von Schleim- und Feststoffen befreit. Zweckmäßig lässt man Fest- und Schleimstoffe absetzen und trennt diese dann nach an sich bekannten Trennungsmethoden, beispielsweise Dekantieren, Separieren und/oder Filtrieren ab. So kann der PMU beispielsweise über eine an sich bekannte Trenneinrichtung, z. B. einen Separator, eine Filtrationsanlage oder einen Sedimentator geleitet werden. Als Trenneinrichtung kann z. B. ein Sandbett dienen, oder es können handelsübliche Separatoren, z. B. Düsen- oder Kammerseparatoren, eingesetzt werden. Gewünschtenfalls können auch eine Mikrofiltrationsanlage oder eine Ultrafiltrationsanlage eingesetzt werden, bei deren Verwendung gleichzeitig eine weitgehende Keim- und Virenfreiheit des filtrierten PMU erreicht werden kann.

Gewünschtenfalls können dem Harn bzw. dem Harnkonzentrat Konservierungsmittel, Germizide, Bakterizide und/oder Antihelmintika zugesetzt werden.

Als vorgereinigtes Harnkonzentrat (iv) kann auch ein aufkonzentriertes PMU Retentat eingesetzt werden, welches aus dem PMU durch eine an sich bekannte Membranfiltration gewonnen werden kann. Der Feststoffgehalt des Retentates und dessen Zusammensetzung können je nach dem eingesetzten PMU und der zur Membranfiltration verwendeten Membran, beispielsweise deren Porenweite, sowie den Bedingungen der Filtration variieren. Beispielsweise kann bei Verwendung einer Nanofiltrationsmembran eine nahezu verlustfreie Konzentration des Östrogengehaltes in dem PMU Retentat bei gleichzeitiger Entfernung von bis zu 50 Gew.% der niedermolekularen PMU Inhaltsstoffe erreicht werden. Für das erfindungsgemäße Verfahren können PMU Retentate eingesetzt werden, welche bis zu einem Verhältnis von ca. 1:10, beispielsweise einem Verhältnis von ca. 1:7 aufkonzentriert wurden und deren Volumen somit bis auf ca. 1/10, beispielsweise ca. 1/7, des ursprünglichen PMU Volumens eingeengt sein kann.

Handelt es sich bei dem, als wässrige Ausgangsphase Verwendung findenden Konzentrat um ein eingeengtes Konzentrat des PMU oder um ein, beispielsweise durch Membranfiltration bereits vorgereinigtes PMU Konzentrat, dann kann das gewonnene Gemisch konjugierter equiner Östrogene, welches durch das erfindungsgemäße Verfahren an nicht-konjugierten lipophilen Verbindungen abgereichert worden ist, schon eine ausreichende Reinheit aufweisen, es kann aber möglicherweise noch signifikante Mengen an phenolischen Harninhaltstoffen enthalten, die durch weitere Verfahrensschritte abgetrennt werden müssen. So kann mit der erhaltenen wässrigen Phase beispielsweise ein Verfahren durchgeführt werden, wie es in den internationalen Patentanmeldungen WO 98/08526 und WO 98/08525 oder in der chinesischen Patentanmeldung CN 1308083 beschrieben worden und dem Fachmann somit aus diesen publizierten Patentanmeldungen geläufig ist, um ein Produkt zu erhalten, welches auch im Hinblick auf den Gehalt an phenolischen Harninhaltsstoffen den erforderlichen pharmazeutischen Spezifikationen für konjugierte Östrogene genügt.

Im Rahmen dieser Erfindung ist die Verwendung einer wässrigen Lösung eines an phenolischen Harninhaltsstoffen bereits abgereicherten natürlichen Gemisches konjugierter Östrogene aus dem Harn trächtiger Stuten oder eines wässrigen Konzentrats eines an phenolischen Harninhaltsstoffen bereits abgereicherten natürlichen Gemisches konjugierter Östrogene aus dem Harn trächtiger Stuten als wässrige Ausgangsphase als besonders bevorzugt anzusehen.

Erfindungsgemäß wird im Verfahrensschritt (a) mit der oben näher bezeichneten wässrigen Ausgangsphase eine Flüssig-Flüssig-Extraktion mit einer ausreichenden Menge eines Extraktionsmittels durchgeführt, welches ein, mit Wasser nicht oder kaum mischbares und für die Extraktion von nicht-konjugierten lipophilen Verbindungen, insbesondere von nicht-konjugierten Flavonoiden, nicht-konjugierten Isoflavonoiden, nicht-konjugierten Norisoprenoiden und nicht-konjugierten Steroiden, geeignetes organisches Lösungsmittel darstellt. Weiterhin soll das organische Lösungsmittel mit der wässrigen Ausgangsphase nicht oder nur kaum mischbar sein, wobei kaum mischbar bedeutet, dass in der wässrigen Phase maximal 6 Vol.-% gelöstes organisches Lösungsmittels vorliegen. Für diesen Flüssig-Flüssig-Extraktionsschritt kann grundsätzlich jedes mit Wasser nicht mischbare organische Lösungsmittel verwendet werden, sofern es die nicht-konjugierten lipophilen Verbindungen aus der wässrigen Phase extrahiert. Für die Extraktion von nicht-konjugierten lipophilen Verbindungen, insbesondere von nicht-konjugierten Flavonoiden, nicht-konjugierten Isoflavonoiden, nicht-konjugierten Norisoprenoiden und nicht-konjugierten Steroiden, geeignet sind beispielsweise folgende organische Lösungsmittel mit 1 bis zu 10 C-Atomen, welche geradkettig, verzweigt oder zyklisch angeordnet sein können: C₄-C₁₀ Alkohole (wie beispielsweise Butanol, Hexanol, Cyclohexanol und Pentanol), C₂-C₁₀ veresterte Säuren (wie beispielsweise Ethylacetat (= Essigsäureethylester), Methylacetat, Propylacetat, Isopropylacetat, Butylacetat, Amylacetat, Ethylmethylmalonat, Dimethylphosphonat), C₃-C₁₀ Aldehyde und C₄-C₁₀ Ketone (wie beispielsweise Butanon, Pentanon, Hexan-2,4-dion, Hexandial, Cyclohexancarbaldehyd, Methylphenylketon und ähnliche), bzw. generell C₃-C₁₀ Alkoxy-Verbindungen, C₂-C₁₀ Ether (Diethylether, Methyl-tertiär-butylether), C₃-C₆ Nitrilen, und C₁-C₃ Halogenalkanen (Methylenchlorid), sowie Mischungen der vorgenannten Lösungsmittel. Insbesondere können Essigsäure-C₁-C₄-alkylester, Hexanol, Diethylether, Methylenchlorid, Methyl-tertiär-butylether und Mischungen der vorgenannten Lösungsmittel als Extraktionsmittel in der vorliegenden Erfindung genutzt werden. Aus dieser Auswahl stellen Essigsäure-C₁-C₄-alkylester, und insbesondere Essigsäureethylester (= Ethylacetat), das bevorzugt zu verwendende Extraktionsmittel der vorliegenden Erfindung dar.

Bei der im Verfahrensschritt (a) erfindungsgemäß durchgeführten Flüssig-Flüssig-Extraktion mit einer ausreichenden Menge eines Extraktionsmittels ist das Volumenverhältnis der wässrigen Ausgangsphase zu dem Extraktionsmittel als nicht limitierend im Rahmen dieser Erfindung zu verstehen. Generell wird ein Volumen an organischem Lösungsmittel eingesetzt, welches dem Volumen der wässrigen Ausgangsphase entspricht, wobei allerdings das Verhältnis von wässriger zu organischer Phase in einem Bereich zwischen 10:1 und 1:10 liegen kann. Vorzugsweise liegt das Volumenverhältnis der wässrigen Ausgangsphase zu dem organischen Extraktionsmittel im Bereich von 5:1 bis 1:3; insbesondere wird ein Volumenverhältnis im Bereich von 2:1 bis 1:2 als zweckmäßig angesehen.

Die Durchführung eines solchen Extraktionsprozess ist dem Fachmann aus dem Stand der Technik bekannt. Üblicherweise wird eine Flüssig-Flüssig-Extraktion in einer Apparatur durchgeführt, die eine kontinuierliche Durchmischung der wässrigen und der mit Wasser nicht mischbaren, organischen Phase ermöglicht. Beispielsweise ist eine sogenannte Mixer-Settler-Apparatur, in der die beiden Phasen durch Rühren vermischt werden, für die Durchführung eines solchen Extraktionsprozess geeignet.

Grundsätzlich ist die erfindungsgemäß beschriebene Flüssig-Flüssig-Extraktion bei jedem pH-Wert der wässrigen Ausgangsphase durchführbar. In einer besonders bevorzugten Variante der erfindungsgemäß beschriebenen Flüssig-Flüssig-Extraktion wird im Verfahrensschritt (a) zunächst der pH-Wert der wässrigen Ausgangsphase auf einen Wert im Bereich zwischen 4 und 12 eingestellt. Vorzugsweise wird der pH-Wert auf einen Wert im Bereich von 4,0 bis 7,0, d.h. im schwach sauren bis neutralen Bereich eingestellt. Ganz besonders bevorzugt wird der pH-Wert auf einen Wert im Bereich von 4,0 bis 6,0, insbesondere im Bereich von 4,7 bis 5,3, eingestellt.

Während der Einstellung des pH-Wertes wird die vorgelegte Lösung zweckmäßigerweise in einem genügend großen, inerten Behälter, wie zum Beispiel in einem Edelstahlfass, mit einem Rührer oder einer vergleichbaren Vorrichtung durchmischt, um so eine schnelle und gleichmäßige Einstellung des pH-Wertes zu gewährleisten. Zur Einstellung des pH-Wertes können übliche Basen oder Säuren eingesetzt werden. So kann beispielsweise zur Absenkung des pH-Wertes eine der üblichen anorganischen oder organischen Säuren, zweckmäßigerweise eine verdünnte Säure, eingesetzt werden. Als besonders geeignet zur Absenkung oder zur Einstellung eines pH-Wertes kleiner als 7 hat sich zum Beispiel die Verwendung von verdünnter Schwefelsäure, vorzugsweise 1 N Schwefelsäure, verdünnter Essigsäure, verdünnter Phosphorsäure oder verdünnter Salzsäure, vorzugsweise 1 N Salzsäure, herausgestellt.

Die erfindungsgemäß beschriebene Flüssig-Flüssig-Extraktion erfordert nicht die Einstellung einer spezifischen Temperatur, sondern kann in einem breiten Temperaturbereich, der zwischen 5 °C und dem Siedepunkt des organischen Lösungsmittels bzw. maximal 95 °C liegen kann, durchgeführt werden. Vorzugsweise wird die erfindungsgemäße Flüssg-Flüssig-Extraktion bei Raumtemperatur durchgeführt, da so der zusätzliche Energiebedarf am geringsten ist. Als Raumtemperatur wird üblicherweise die Umgebungstemperatur angesehen; beispielsweise wird so eine Temperatur bezeichnet, die zwischen 10° und 30 °C liegt.

Die Zeitdauer einer solchen Flüssig-Flüssig-Extraktion wird im Rahmen dieser Erfindung als nicht limitierend angesehen und kann zwischen 5 min und mehreren Stunden liegen; die Zeitdauer schwankt in Abhängigkeit von der Menge der eingesetzten wässrigen Ausgangsphase. Typischerweise werden die wässrige Phase aus Verfahrensschritt (a) und das organische Extraktionsmittel für 5 bis 60 min, vorzugsweise für 10 bis 20 min, miteinander vermischt, um einen möglichst vollständigen Übergang der nicht-konjugierten lipophilen Bestandteile aus der wässrigen in die organische Phase zu erzielen.

Im Anschluss an den Extraktionsschritt durch Durchmischung lässt man das Phasengemisch stehen, um eine Trennung der Phasen zu erreichen. Die Phasentrennung kann je nach den eingesetzten Volumina einen Zeitraum von 10 min bis zu mehreren Stunden einnehmen, vorzugsweise lässt man die Phasen 30 bis 120 min stehen. Wenn sich die wässrige Phase und die organische Phase voneinander separiert haben, wird die wässrige Phase abgetrennt und zur Weiterverwendung aufgehoben, während die organische Phase verworfen wird.

Der oben beschriebene Verfahrensschritt (a) kann gegebenenfalls wiederholt werden; dann schließt sich erfindungsgemäß ein optionaler Verfahrensschritt (b) an, in dem mit der aus dem Verfahrensschritt (a) erhaltenen wässrigen Phase eine erneute Flüssig-Flüssig-Extraktion mit einer ausreichenden Menge eines Extraktionsmittels, welches ein, für die Extraktion von nicht-konjugierten lipophilen Verbindungen, insbesondere von nicht-konjugierten Flavonoiden, nicht-konjugierten Isoflavonoiden, nicht-konjugierten Norisoprenoiden und nicht-konjugierten Steroiden, geeignetes, mit Wasser nicht oder kaum mischbares organisches Lösungsmittel darstellt, durchgeführt wird.

Für die Auswahl des Extraktionsmittels und die Art der Durchführung der Extraktion, d.h., die Verwendung findende Apparatur, die Dauer und die Temperatur des Extraktionsprozesses, sollen die oben unter Verfahrensschritt (a) ausgeführten Möglichkeiten als exemplarisch gelten.

In den Verfahrensschritten (a) und (b) können erfindungsgemäß in beiden Schritten sowohl unterschiedliche Extraktionsmittel als auch das gleiche Extraktionsmittel verwendet werden. Vorzugsweise wird in beiden Extraktionsschritten das gleiche Extraktionsmittel verwendet. Insbesondere soll in beiden Extraktionsschritten Essigsäure-C₁-C₄-alkylester, insbesondere aber Essigsäureethylester, als Extraktionsmittel Verwendung finden.

Bei der im Verfahrensschritt (b) erfindungsgemäß durchgeführten Flüssig-Flüssig-Extraktion mit einer ausreichenden Menge eines Extraktionsmittels ist das Volumenverhältnis der bereits einmal extrahierten, die konjugierten equinen Östrogene enthaltenden, wässrigen Phase aus Verfahrensschritt (a) zu dem Extraktionsmittel als nicht limitierend im Rahmen dieser Erfindung zu verstehen. Generell wird ein Volumen an organischem Lösungsmittel eingesetzt, welches deutlich unter dem Volumen der aus Verfahrensschritt (a) erhaltenen wässrigen Phase liegt, wobei allerdings das Verhältnis von wässriger zu organischer Phase in einem Bereich zwischen 40:1 und 1:2 liegen kann. Vorzugsweise liegt das Volumenverhältnis der aus Verfahrensschritt (b) erhaltenen wässrigen Phase zu dem organischen Extraktionsmittel im Bereich von 20:1 bis 1:1; insbesondere wird ein Volumenverhältnis im Bereich von 10:1 bis 2:1 als zweckmäßig angesehen.

Im Anschluss an den Extraktionsschritt durch Durchmischung im Verfahrensschritt (b) lässt man das Phasengemisch stehen, um eine Trennung der Phasen zu erreichen. Die Phasentrennung kann einen Zeitraum von 10 min bis zu mehreren Stunden einnehmen, vorzugsweise lässt man die Phasen 20 bis 90 min stehen. Wenn sich die wässrige Phase und die organische Phase voneinander separiert haben, wird die wässrige Phase abgetrennt und zur Weiterverwendung aufgehoben, während die organische Phase verworfen wird.

Nach der Trennung der organischen Phase von der wässrigen Phase wird im Verfahrensschritt (c) eine, das natürliche Gemisch konjugierter Östrogene enthaltende wässrige Phase gewonnen. Diese wässrige Phase enthält das im PMU auftretende natürliche Gemisch konjugierter Östrogene neben einem nur äußerst geringen Anteil des, ursprünglich im PMU oder den aufbereiteten PMU-Konzentraten vorhandenen Gehaltes an nicht-konjugierten lipophilen Bestandteilen. Gewünschtenfalls kann diese wässrige Phase auf an sich bekannte Weise weiter eingeengt werden, um ein weitgehend von organischem Lösungsmittel befreites, zur Weiterverarbeitung geeignetes Konzentrat zu erhalten. So können beispielsweise aus der erhaltenen wässrigen Phase die noch vorhandenen Reste an organischem Lösungsmittel abdestilliert werden. Durch die Destillation kann auch der Trockensubstanzgehalt der wässrigen Extraktphase auf einen konkreten Wert, vorzugsweise auf einen Trockensubstanzgehalt im Bereich zwischen 5 und 15 %, insbesondere auf einen Trockensubstanzgehalt von 9 %, eingestellt werden. Im Anschluss kann zur Stabilisierung des gewonnenen natürlichen Gemisches konjugierter equiner Östrogene noch der pH-Wert der wässrigen Extraktlösung auf einen Wert im alkalischen Bereich, vorzugsweise im Bereich zwischen 8 und 13, vorzugsweise auf einen Wert zwischen 9 und 12, eingestellt werden. Zur Einstellung des pH-Wertes eignen sich üblicherweise für die pH-Wert Einstellung Verwendung findende Basen, beispielsweise 1 N NaOH oder Na₂CO₃.

Die erfindungsgemäß im Verfahrensschritt (c) gewonnene und gegebenenfalls noch weiter aufgearbeitete oder konzentrierte wässrige Phase kann als Ausgangsmaterial für die Herstellung von, das natürliche Gemisch konjugierter equiner Östrogene enthaltenden Arzneimitteln dienen. Gewünschtenfalls kann durch einen geeigneten Trocknungsprozess wie beispielsweise Sprühtrocknung ein elutionsmittelfreies Feststoffgemisch hergestellt werden. Sofern das natürliche Gemisch konjugierter Östrogene für die Herstellung fester Arzneimittel verwendet werden soll, kann es zweckmäßig sein, der die konjugierten Östrogene enthaltenden wässrigen Phase einen festen Trägerstoff bereits vor einer Konzentrierung oder Trocknung zuzumischen, um auf diese Weise ein die konjugierten Östrogene und Trägerstoffe enthaltendes Feststoffgemisch zu erhalten. Sowohl die das Östrogengemisch enthaltende wässrige Phase als auch ein daraus hergestelltes Konzentrat oder getrocknetes Feststoffprodukt können in an sich bekannter Weise in feste oder flüssige galenische Zubereitungen wie beispielsweise Tabletten, Dragees, Kapseln oder Emulsionen eingearbeitet werden. Diese galenischen Zubereitungen können nach an sich bekannten Methoden hergestellt werden unter Verwendung üblicher fester oder flüssiger Trägerstoffe wie z. B. Stärke, Cellulose, Milchzucker oder Talkum oder flüssigen Paraffinen und/oder unter Verwendung von üblichen pharmazeutischen Hilfsstoffen, beispielsweise Tablettensprengmitteln, Lösungsvermittlern oder Konservierungsmitteln. So kann das die konjugierten Östrogene enthaltende Produkt mit den pharmazeutischen Träger- und Hilfsstoffen auf an sich bekannte Weise vermischt werden und das Gemisch in eine geeignete Dosierungsform überführt werden.

Bei der erfindungsgemäß beschriebenen Flüssig-Flüssig-Extraktion werden aus einer wässrigen Ausgangsphase, welche entweder eine wässrige Lösung eines an phenolischen Harninhaltsstoffen bereits abgereicherten natürlichen Gemisches konjugierter Östrogene aus dem Harn trächtiger Stuten, ein wässriges Konzentrat eines an phenolischen Harninhaltsstoffen bereits abgereicherten natürlichen Gemisches konjugierter Östrogene aus dem Harn trächtiger Stuten, ein Konzentrat einer Harnflüssigkeit, oder ein, gegebenenfalls durch Filtration vorgereinigtes Harnkonzentrat darstellen kann, eine Vielzahl nicht-konjugierter lipophiler Verbindungen in einem einfachen Verfahren entfernt. Bei den abgetrennten nicht-konjugierten lipophilen Verbindungen handelt es sich insbesondere um nicht-konjugierte Flavonoide, nicht-konjugierte Isoflavonoide, nicht-konjugierte Norisoprenoide und nicht-konjugierte Steroide, hierbei insbesondere um nicht-konjugierte Androstan- und nicht-konjugierte Pregnan-Derivate.

Im Vergleich zu den klassischen Flüssig-Flüssig-Extraktionsmethoden finden hier geringere Volumina organischer Lösungsmittel Verwendung, da als wässrige Ausgangsphase stets ein Konzentrat des ursprünglichen PMU eingesetzt wird. Findet beispielsweise für die wässrige Ausgangsphase der erfindungsgemäß beschriebenen Flüssig-Flüssig-Extraktion ein wässriges Konzentrat Verwendung, welches anhand dem in der internationalen Patentanmeldung WO 98/08526 beschriebenen Verfahren gewonnen worden ist, werden anstelle von 5000 I PMU nur ca. 35 I Konzentrat und entsprechend geringe Mengen organischer Lösungsmittel für die Extraktion eingesetzt.

Wird für die erfindungsgemäße Flüssig-Flüssig-Extraktion als wässrige Ausgangsphase eine wässrige Lösung eines an phenolischen Harninhaltsstoffen bereits abgereicherten natürlichen Gemisches konjugierter Östrogene aus dem Harn trächtiger Stuten oder ein wässriges Konzentrat eines an phenolischen Harninhaltsstoffen bereits abgereicherten natürlichen Gemisches konjugierter Östrogene aus dem Harn trächtiger Stuten eingesetzt, zeichnet sich das als Wirkstoff-Extrakt gewonnene, an nicht-konjugierten lipophilen Bestandteilen und an phenolischen Harninhaltstoffen abgereicherte natürliche Gemisch konjugierter equiner Östrogene durch einen deutliche Optimierung der pharmazeutischen Spezifikation aus, wie erfindungsgemäß festgestellt wurde. Insbesondere tritt durch die Flüssig-Flüssig-Extraktion eine 8 bis 20%ige Verbesserung des Verhältnisses der konjugierten equinen Östrogene zur Trockensubstanz auf, ohne dass bei dem Extraktionsverfahren nennenswerte Verluste an konjugierten equinen Östrogenen zu beobachten sind. Damit weist die im Verfahrensschritt (c) gewonnene wässrige Phase, welche das natürliche Gemisch konjugierter equiner Östrogene enthält, im Vergleich zum Stand der Technik eine vorteilhafte Zusammensetzung und einen, bezogen auf die Trockensubstanz erhöhten Gesamthormongehalt auf. Dadurch wird ein z. B. in bezug auf die Zusammensetzung und den Wirkstoff-Gehalt deutlich verbessertes Qualitätsprodukt erhalten.

Es muss als ausgesprochen überraschend bezeichnet werden, dass ein vermeintlich einfaches Verfahren einer Flüssig-Flüssig-Extraktion einer wässrigen Lösung oder Konzentrats eines an phenolischen Harninhaltsstoffen abgereicherten natürlichen Gemisches konjugierter Östrogene aus dem, unterschiedliche und wechselnde Mengen nicht-konjugierte lipophiler Bestandteile enthaltenden Harn trächtiger Stuten derart zur Qualitätsverbesserung des gewonnenen Wirkstoffextraktes beiträgt. Insbesondere ist es sehr überraschend, dass der Anteil an nicht-konjugierten lipophilen Verbindungen, der in Abhängigkeit vom verwendeten PMU sowohl mengenmäßig als auch in der Zusammensetzung stark schwanken kann, durch das erfindungsgemäße Verfahren derart sicher verringert werden kann, dass im Verfahrensschritt (c) als wässrige Phase ein Gemisch natürlicher konjugierter equiner Östrogene erhalten werden kann, welches die hohen Anforderungen an die pharmazeutische Spezifikation, beispielsweise die gemäß der USP oder der Europäischen Pharmacopeia aufgestellten Anforderungen, erfüllt.

Weiterhin muss als sehr überraschend angesehen werden, dass bei der Durchführung des erfindungsgemäßen Extraktionsverfahrens auch solche nichtkonjugierten lipophilen Verbindungen, wie beispielsweise entschäumende Agenzien, abgetrennt werden, welche dem PMU zuvor als Hilfsstoffe in vorgeschalteten Verarbeitungsschritten, beispielsweise im Rahmen der Konzentratherstellung, zugesetzt worden sind.

Es hat sich beim erfindungsgemäßen Verfahren als weiterer Vorteil herausgestellt, dass die Weiterverarbeitung des gewonnenen extrahierten Konzentrats in Form eines an nicht-konjugierten lipophilen Verbindungen abgereicherten natürlichen Gemisches konjugierter equiner Östrogene und seine Überführung in eine galenische Form wesentlich vereinfacht und erleichtert wird. Überraschenderweise zeichnet sich der, mit dem erfindungsgemäßen Verfahren gewonnene Wirkstoffextrakt durch ein sehr gutes Trocknungsverhalten und der, nach der Trocknung erhaltene Feststoff durch eine außerordentlich gute Fließfähigkeit aus. So lässt sich beispielsweise das erfindungsgemäß erhaltene, extrahierte Konzentrat deutlich einfacher auf Trägermaterial auftragen als eine nicht extrahierte Lösung: Auch die Einstellung der Wirkstoffkonzentration wird vereinfacht und reproduzierbar.

Das erfindungsgemäße Verfahren bietet, wie vorstehend bereits im Detail beschrieben, eine Reihe von Vorteilen und Verbesserungen gegenüber dem Stand der Technik. So ermöglicht die Erfindung die Verwendung auch von wechselnde Mengen nicht-konjugierter lipophiler Bestandteile enthaltendem PMU, der beispielsweise einen erhöhten Anteil an freien Flavonoiden, freien Isoflavonoiden, freien Norisoprenoiden oder freien Steroid-Derivaten, hier insbesondere an freien Androstan- oder Pregnan-Steroiden, aufweisen darf, ohne dass einzuhaltende pharmazeutische Spezifikationen gefährdet werden. Mit dem erfindungsgemäßen Verfahren kann eine gleichmäßige Zusammensetzung einzelner Extraktchargen gewährleistet werden, da die nicht-konjugierten lipophilen Bestandteile, deren Gehalt und Zusammensetzung im PMU in Abhängigkeit von der Art der aufgenommenen Nahrung der trächtigen Stuten variieren kann, stets eliminiert werden und damit die erhaltenen Extrakte alle einen vergleichbaren Gehalt konjugierter equiner Östrogene bezogen auf die Trockensubstanz aufweisen. Weiterhin wird durch die mit dem erfindungsgemäßen Verfahren erzielte Abtrennung der nicht-konjugierten lipophilen Bestandteile eine höhere Konzentration der Wirksubstanzen, d.h. der konjugierten equinen Östrogene, im erhaltenen Extrakt erreicht. Das erfindungsgemäße Verfahren weist zusätzlich auch wirtschaftliche Vorteile auf, da die Gefahr, wertvolle Wirkstoffe bei Nichteinhaltung der pharmazeutischen Spezifikation zu verlieren, beispielsweise bei nicht ausreichenden Gehalten konjugierter Östrogene bezogen auf die Trockensubstanz, deutlich vermindert ist. Weiterhin wird durch die Anwendung des erfindungsgemäß beschriebenen Verfahrens eine wesentlich genauere und reproduzierbarere Einstellung des Wirkstoffgehaltes des erhaltenen Extraktes ermöglicht. Das erfindungsgemäße Verfahren liefert eine qualitätsverbesserte Wirkstoffkomponente mit einem auf den Trockensubstanz-Gehalt bezogenen erhöhten Hormongehalt. Diese Wirkstoffkomponente eignet sich hervorragend für die Herstellung von Pharmazeutika, die als Wirkstoff ein Gemisch natürlicher konjugierter equiner Östrogene enthalten.

Die nachfolgenden Beispiele sollen die Erfindung näher erläutern, ohne jedoch ihren Umfang zu beschränken.

### Beispiele

In nachfolgenden Beispielen wird eine allgemeine Arbeitsvorschrift zur Gewinnung von Wirkstoff-Extrakten aus PMU angegeben, die das natürliche Gemisch der im PMU enthaltenen konjugierten Östrogene enthalten und an nicht-konjugierten lipophilen Verbindungen, wie beispielsweise nicht-konjugierte Flavonoide, nicht-konjugierte Isoflavonoide, nicht-konjugierte Norisoprenoide, nicht-konjugierte Steroide, insbesondere Androstan- und Pregnan-Steroide, und vergleichbare nicht-konjugierte Verbindungen, weitgehend abgereichert sind. Es wird gezeigt, wie sich erfindungsgemäß auch aus PMU, der wechselnde bzw. erhöhte Anteile an nicht-konjugierten lipophilen Verbindungen aufweisen kann, ein Qualitätsextrakt mit hohen Wirkstoffgehalten gewinnen lässt.

### Extraktion eines wässrigen Konzentrates eines an phenolischen Harninhaltsstoffen bereits abgereicherten natürlichen Gemisches konjugierter Östrogene aus dem Harn trächtiger Stuten

Als wässrige Ausgangsphase finden 35,3 kg (Beispiel 1) bzw. 26,7 kg (Beispiel 2) wässriges Konzentrat eines an phenolischen Harninhaltsstoffen bereits abgereicherten natürlichen Gemisches konjugierter Östrogene aus dem Harn trächtiger Stuten Verwendung, welche mit Hilfe des in der internationalen Patentanmeldung WO 98/08526 beschriebenen Verfahrens aus ca. 5000 I PMU hergestellt wurden (Trockensubstanzgehalt (= TS) und auch mittels HPLC und GC bestimmte Gehalte an konjugierten Östrogenen, beispielsweise Estronsulfatsalz, und nicht-konjugierten lipophilen Verbindungen, beispielsweise Formononetin, sind in der nachfolgenden Beispieltabelle für verschiedene Chargen an eingesetztem wässrigen Konzentrat dargestellt). Dieses wässrige Konzentrat wird im Edelstahlfass mit Hilfe eines Rührers durchmischt, während der pH-Wert mit 1N H₂SO₄ auf einen Wert von circa 5,0 eingestellt wird. Die erhaltene Lösung wird in einer Mixer-Settler Apparatur im Verhältnis 10:8 wässrige Phase zu organischer Phase mit Essigsäureethylester (EE - Mengenangaben sieh folgende Beispieltabelle) versetzt und circa 15 min stark gerührt. Danach lässt man das Gemisch zur Trennung der Phasen ca. 90 min stehen. Im Anschluss werden die Phasen getrennt und die wässrige Phase wird erneut im Verhältnis 10:2 wässrige Phase zu organischer Phase mit Essigsäureethylester (EE - Mengenangaben sieh folgende Beispieltabelle) versetzt und 15 min gerührt. Nach der Extraktion lässt man das Gemisch zur Phasentrennung circa 30 min stehen. Nach der Abtrennung der organischen Phase wird die wässrige Phase in einen Reaktionskessel übergeführt. Aus der wässrigen Phase werden unter Normaldruck noch vorhandene Reste an Essigsäureethylester abdestilliert. Durch die Destillation wird der Trockensubstanzgehalt auf ca. 9 % eingestellt. Im Anschluss wird der pH-Wert der Lösung durch Zugabe von 1N NaOH oder Na₂CO₃ auf circa 11,0 eingestellt. Der Gehalt der so erhaltenen wässrigen Extraktphase an konjugierten Östrogenen wird mittels HPLC und GC Analysen überprüft (Trockensubstanzgehalt (= TS) und auch mittels HPLC und GC bestimmte Gehalte an konjugierten Östrogenen, beispielsweise Estronsulfatsalz, und nicht-konjugierten lipophilen Verbindungen, beispielsweise Formononetin, sind in der nachfolgenden Beispieltabelle für verschiedene Chargen an eingesetztem wässrigen Konzentrat dargestellt). Das Verhältnis der einzelnen Hormonbestandteile zueinander, d.h. der relative Anteil von Estronsulfat, Equilinsulfat und 17-alpha-DH-Equilinsulfat im Gemisch konjugierter Östrogene, kann in den einzelnen Chargen aufgrund der natürlichen Schwankungen im PMU variieren. Durch gezieltes Mischen einzelner Chargen miteinander kann das gewünschte Verhältnis der einzelnen Hormonbestandteile zueinander eingestellt werden.

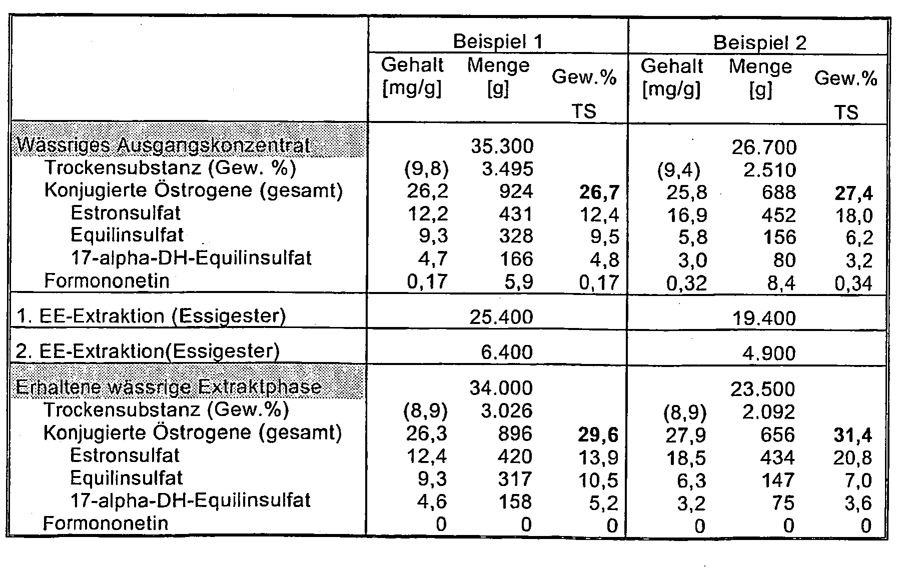

Dieses Extraktionsverfahrens kann in analoger Weise durchgeführt werden, wenn anstelle des wässrigen Konzentrates eines an phenolischen Harninhaltsstoffen bereits abgereicherten natürlichen Gemisches konjugierter Östrogene aus dem Harn trächtiger Stuten als wässrige Ausgangsphase entweder ein Konzentrat einer Harnflüssigkeit, ein, gegebenenfalls durch Filtration vorgereinigtes Harnkonzentrat, oder eine wässrige Lösung eines an phenolischen Harninhaltsstoffen bereits abgereicherten natürlichen Gemisches konjugierter Östrogene aus dem Harn trächtiger Stuten, als wässrige Ausgangsphase eingesetzt werden.

## Patentansprüche

1. Verfahren zur Gewinnung eines natürlichen Gemisches konjugierter equiner Östrogene, **dadurch gekennzeichnet, dass** das gewonnene Gemisch abgereichert ist an nicht-konjugierten lipophilen Verbindungen aus der Gruppe umfassend nicht-konjugierte Flavonoide, nicht-konjugierte Isoflavonoide, nicht-konjugierte Norisoprenoide, nicht-konjugierte Steroide, insbesondere Androstan- und Pregnan-Steroide, und vergleichbare nicht-konjugierte Verbindungen, und dass das Verfahren sich **dadurch** auszeichnet, dass man
a) eine wässrige Ausgangsphase, ausgewählt aus der Gruppe umfassend
(i) eine wässrige Lösung eines an phenolischen Harninhaltsstoffen bereits abgereicherten natürlichen Gemisches konjugierter Östrogene aus dem Harn trächtiger Stuten,
(ii) ein wässriges Konzentrat eines an phenolischen Harninhaltsstoffen bereits abgereicherten natürlichen Gemisches konjugierter Östrogene aus dem Harn trächtiger Stuten,
(iii) ein durch Einengen gewonnenes Konzentrat einer Harnflüssigkeit, und
(iv) ein von Fest- und Schleimstoffen befreites Harnkonzentrat, welches durch Trennverfahren ausgewählt aus der Gruppe umfassend Dekantieren, Separieren, Filtrieren und Kombinationen der vorgenannten Verfahren vorgereinigt wurde, oder ein durch Membranfiltration gewonnenes aufkonzentriertes Harn-Retentat,
einer Flüssig-Flüssig Extraktion mit einer ausreichenden Menge eines Extraktionsmittels unterwirft und im Anschluß die wäßrige Phase abtrennt, wobei das Extraktionsmittel ein, für die Extraktion von nicht-konjugierten lipophilen Verbindungen aus oben angegebener Gruppe geeignetes, mit Wasser nicht oder kaum mischbares organisches Lösungsmittel darstellt und ausgewählt ist aus der Gruppe bestehend aus geradkettigen, verzweigten oder zyklischen C₄-C₁₀ Alkoholen, C₂-C₁₀ veresterten Säuren, C₃-C₁₀ Aldehyden, C₄-C₁₀ Ketonen, C₂-C₁₀ Ethern, C₃-C₆ Nitrilen, und C₁-C₃ Halogenalkanen, und Mischungen der vorgenannten Lösungsmittel, und
b) gegebenenfalls mit der erhaltenen wässrigen Phase den Verfahrensschritt (a) wiederholt, und
c) eine, das natürliche Gemisch konjugierter Östrogene enthaltende wässrige Phase gewinnt und diese gegebenenfalls einengt.

2. Verfahren nach Anspruch 1, worin im Verfahrensschritt (a) als wässrige Ausgangsphase eine wässrige Lösung (i) eines an phenolischen Harninhaltsstoffen bereits abgereicherten natürlichen Gemisches konjugierter Östrogene aus dem Harn trächtiger Stuten, oder ein wässriges Konzentrat (ii) eines an phenolischen Harninhaltsstoffen bereits abgereicherten natürlichen Gemisches konjugierter Östrogene aus dem Harn trächtiger Stuten verwendet wird.

3. Verfahren nach Anspruch 2, wobei die Abreicherung an phenolischen Harninhaltsstoffen durch Festphasen-Extraktion an nicht-ionischen semipolaren Adsorberharzen oder an hydrophobisiertem Kieselgel erfolgt.

4. Ein Verfahren nach einem der vorherigen Ansprüche, worin das Extraktionsmittel ausgewählt ist aus der Gruppe bestehend aus Essigsäure-C₁-C₄-alkylester, Hexanol, Diethylether, Methylenchlorid, Methyl-tertiär-butylether und Mischungen der vorgenannten Lösungsmittel.

5. Ein Verfahren nach Anspruch 4, worin das Extraktionsmittel Essigsäure-C₁-C₄-alkylester, vorzugsweise Essigsäureethylester, ist.

6. Ein Verfahren nach einem der Ansprüche 1 bis 5, worin im Verfahrensschritt (a) die wässrige Ausgangsphase auf einen pH-Wert im Bereich zwischen 4 und 12 eingestellt ist.

7. Ein Verfahren nach Anspruch 6, worin der pH-Wert im Bereich von 4,0 bis 7,0, insbesondere im Bereich von 4,0 bis 6,0, und vorzugsweise im Bereich von 4,7 bis 5,3, eingestellt ist.

8. Ein Verfahren nach einem der Ansprüche 1 bis 7, worin im Verfahrensschritt (a) das Volumenverhältnis der wässrigen Ausgangsphase zu dem Extraktionsmittel im Bereich von 5:1 bis 1:3, vorzugsweise im Bereich von 2:1 bis 1:2, liegt.

9. Ein Verfahren nach einem der Ansprüche 1 bis 8, worin im Verfahrensschritt (b) das Volumenverhältnis der aus Verfahrensschritt (a) erhaltenen wässrigen Phase zu dem Extraktionsmittel im Bereich von 20:1 bis 1:1, vorzugsweise im Bereich von 10:1 zu 2:1, liegt.

## Claims

1. A method of recovering a natural mixture of conjugated equine estrogens, **characterized in that** the recovered mixture is depleted in non-conjugated lipophilic compounds from the group comprising non-conjugated flavonoids, non-conjugated isoflavonoids, non-conjugated norisoprenoids, non-conjugated steroids, in particular androstane and pregnane steroids, and comparable non-conjugated compounds, and **in that** the method comprises
a) an aqueous starting phase selected from the group comprising
(i) an aqueous solution of a natural mixture of pregnant mares' urine conjugated estrogens which is already depleted in phenolic urine contents,
(ii) an aqueous concentrate of a natural mixture of pregnant mares' urine conjugated estrogens which is already depleted in phenolic urine contents,
(iii) an evaporatively obtained concentrate of a urine liquid, and
(iv) a urine concentrate freed of solids and mucilaginous substances, which was prepurified by separation processes selected from the group comprising decanting, separation, filtering and combinations thereof, or a concentrated urine retentate obtained by membrane filtration,
being subjected to a liquid-liquid extraction with a sufficient quantity of an extracting agent and the aqueous phase being subsequently separated off, the extracting agent constituting an organic solvent which is at most only slightly miscible with water and is suitable for the extraction of non-conjugated lipophilic compounds from the above-indicated group, and being selected from the group consisting of straight-chain, branched or cyclic C₄-C₁₀ alcohols, C₂-C₁₀ esterified acids, C₃-C₁₀ aldehydes, C₄-C₁₀ ketones, C₂-C₁₀ ethers, C₃-C₆ nitriles and C₁-C₃ haloalkanes, and mixtures thereof, and
b) optionally repeating step (a) with the aqueous phase obtained, and
c) an aqueous phase containing the natural mixture of conjugated estrogens being recovered and optionally concentrated by evaporation.

2. Method according to Claim 1, wherein step (a) neutralizes as aqueous starting phase an aqueous solution (i) of a natural mixture of pregnant mares' urine conjugated estrogens which is already depleted in phenolic urine contents, or an aqueous concentrate (ii) of a natural mixture of pregnant mares' urine conjugated estrogens which is already depleted in phenolic urine contents.

3. Method according to Claim 2, wherein the depleting in phenolic urine contents is effected by solid phase extraction on non-ionic semipolar adsorbent resins or on hydrophobicized silica gel.

4. Method according to any one of the preceding claims, wherein the extracting agent is selected from the group consisting of C₁-C₄-alkyl acetate, hexanol, diethyl ether, methylene chloride, methyl tertiarybutyl ether and mixtures thereof.

5. Method according to Claim 4, wherein the extracting agent is C₁-C₄-alkyl acetate, preferably ethyl acetate.

6. Method according to any one of Claims 1 to 5, wherein the aqueous starting phase in step (a) is adjusted to a pH in the range between 4 and 12.

7. Method according to Claim 6, wherein the pH is in the range from 4.0 to 7.0, in particular in the range from 4.0 to 6.0 and preferably in the range from 4.7 to 5.3.

8. Method according to any one of Claims 1 to 7, wherein the volume ratio of the aqueous starting phase to the extracting agent lies in the range from 5:1 to 1:3 and preferably in the range from 2:1 to 1:2 in step (a).

9. Method according to any one of Claims 1 to 8, wherein the volume ratio of the aqueous phase obtained from step (a) to the extracting agent lies in the range from 20:1 to 1:1 and preferably in the range from 10:1 to 2:1 in step (b).

## Revendications

1. Procédé de production d'un mélange naturel d'estrogènes équins conjugués, **caractérisé en ce que** le mélange obtenu est appauvri en composés lipophiles non conjugués du groupe comprenant les flavonoïdes non conjugués, les isoflavonoïdes non conjugués, les norisoprénoïdes non conjugués, les stéroïdes non conjugués, en particulier les stéroïdes de type androstane et prégnane, et des composés non conjugués comparables et le procédé se **caractérise en ce qu'**on soumet
a) une phase de départ aqueuse, choisie dans le groupe comprenant
(i) une solution aqueuse d'un mélange naturel d'estrogènes conjugués déjà appauvri en constituants urinaires phénoliques de l'urine de juments pleines,
(ii) un concentrat aqueux d'un mélange naturel d'estrogènes conjugués déjà appauvri en constituants urinaires phénoliques de l'urine de juments pleines,
(iii) un concentrat d'un liquide urinaire obtenu par concentration, et
(iv) un concentrat urinaire libéré des solides et substances muqueuses qui a été prépurifié par un procédé de séparation choisi dans le groupe comprenant la décantation, la séparation, la filtration et les combinaisons des procédés susmentionnés, ou un rétentat d'urée concentré obtenu par filtration membranaire,
à une extraction liquide-liquide avec une quantité suffisante d'un agent d'extraction puis on sépare la phase aqueuse, l'agent d'extraction étant un solvant organique approprié pour l'extraction de composés lipophiles non conjugués du groupe indiqué ci-dessus, non miscible ou quasiment non miscible avec l'eau et étant choisi dans le groupe constitué par les alcools en C₄-C₁₀ linéaires, ramifiés ou cycliques, les acides estérifiés en C₂-C₁₀, les aldéhydes en C₃-C₁₀, les cétones en C₄-C₁₀, les éthers en C₂-C₁₀, les nitriles en C₃-C₆, et les halogénoalcanes en C₁-C₃, et les mélanges des solvants susmentionnés, et
b) le cas échéant, on répète l'étape de procédé (a) avec la phase aqueuse obtenue, et
c) on obtient une phase aqueuse contenant le mélange naturel d'estrogènes conjugués et celle-ci est le cas échéant concentrée.

2. Procédé selon la revendication 1, dans lequel, dans l'étape de procédé (a), on utilise comme phase de départ aqueuse une solution aqueuse (i) d'un mélange naturel d'estrogènes conjugués déjà appauvri en constituants urinaires phénoliques de l'urine de juments pleines, ou un concentrat aqueux (ii) d'un mélange naturel d'estrogènes conjugués déjà appauvri en constituants urinaires phénoliques de l'urée de juments pleines.

3. Procédé selon la revendication 2, dans lequel l'appauvrissement en constituants urinaires phénoliques est réalisé par extraction en phase solide sur des résines absorbantes semi-polaires non ioniques ou sur du gel silicique hydrofugé.

4. Procédé selon l'une quelconque des revendications précédentes, dans lequel l'agent d'extraction est choisi dans le groupe constitué par les esters alkyliques en C₁-C₄ de l'acide acétique, l'hexanol, le diéthyléther, le chlorure de méthylène, le méthyl-tert-butyléther et les mélanges des solvants susmentionnés.

5. Procédé selon la revendication 4, dans lequel l'agent d'extraction est l'ester alkylique en C₁-C₄ de l'acide acétique, de préférence l'ester éthylique de l'acide acétique.

6. Procédé selon l'une quelconque des revendications 1 à 5, dans lequel, dans l'étape de procédé (a), la phase de départ aqueuse est réglée à un pH dans la plage entre 4 et 12.

7. Procédé selon la revendication 6, dans lequel le pH est réglé dans la plage de 4,0 à 7,0, en particulier dans la plage de 4,0 à 6,0, et de préférence dans la plage de 4,7 à 5,3.

8. Procédé selon l'une quelconque des revendications 1 à 7, dans lequel, dans l'étape de procédé (a), le rapport volumique de la phase aqueuse de départ à l'agent d'extraction se situe dans la plage de 5:1 à 1:3, de préférence dans la plage de 2:1 à 1:2.

9. Procédé selon l'une quelconque des revendications 1 à 8, dans lequel, dans l'étape de procédé (b), le rapport volumique de la phase aqueuse obtenue de l'étape de procédé (a) à l'agent d'extraction se situe dans la plage de 20:1 à 1:1, de préférence dans la plage de 10:1 à 2:1.
